# EUROPEAN PATENT APPLICATION

(11) **EP 4 682 895 A1**
(43) Date of publication of application: **21.01.2026**
(21) Application number: 25189724.5
(22) Date of filing: 15.07.2025
(51) Int. Cl.: G16H 10/20, G16H 50/20, G16H 70/20

(54) **SYSTEM AND METHOD FOR GENERATING RELATED DOCUMENTS AND DATA ON THE BASIS OF CLINICAL PROTOCOL USING LARGE LANGUAGE MODEL**

(30) Priority: 16.07.2024 KR 20240093596
(71) Applicant: JNPMEDI Inc., Incheon 21998 (KR)
(72) Inventor: PARK, Young Yong, 22003 Incheon (KR)
(74) Representative: Vossius & Partner Patentanwälte Rechtsanwälte mbB

(57) **Abstract**

The present invention relates to a system for generating related documents and data on the basis of a clinical protocol using a large language model, and the system comprises: a service provision module for receiving a clinical protocol from at least one user terminal, generating documents and/or data related to the clinical protocol, and providing the documents and/or data to the at least one user terminal; and a large language model for receiving at least one prompt and information related to the clinical protocol indexed by the service provision module from the service provision module, and providing an output thereof to the service provision module, wherein the service provision module includes: a conversion rule management unit for providing a conversion rule generation function including the at least one prompt to the user terminal; and a conversion execution unit for providing the conversion rule and the clinical protocol to the large language model, and providing documents and/or data output from the large language model to the at least one user terminal in a final output form.

## Description

### BACKGROUND OF THE INVENTION

### Field of the Invention

The present invention relates to a system and method for generating related documents and data on the basis of a clinical protocol using a large language model.

### Background of the Related Art

In the process of conducting a clinical trial, it is essential to write or record various documents and data, and these documents and data are generally written on the basis of the contents described in the clinical protocol. However, since the amount of documents and data that should be written in the clinical trial process is enormous, the time and cost required for generating these documents and data occupy a large portion of work, and with the trend of digitizing the work documents, documents related to clinical trials are also generated and written as electronic documents.

Meanwhile, generative AI techniques using a large language model are developed recently with the advancement in technology, and the trend of utilizing the generative AI techniques in works is also increasing steadily. However, even when various contents are generated using a large language model, large language models have the problem of hallucination, such as giving incorrect answers as they learn public open general data, and demands on techniques capable of deriving more accurate results are continued.

### SUMMARY OF THE INVENTION

Accordingly, the present invention has been made in view of the above-mentioned problems occurring in the prior art, and it is an object of the present invention to provide a system and method for generating related documents and data on the basis of a clinical protocol using a large language model, which can reduce the time and cost required for generating documents and data needed for conducting a clinical trial by generating various documents and data needed to conduct a clinical trial using a large language model on the basis of previously generated clinical protocols.

The technical problems of the present invention are not limited to the technical problems mentioned above, and unmentioned other technical problems can be clearly understood by those skilled in the art from the following description.

To accomplish the object, according to an aspect of the present invention, there is provided a system for generating related documents and data on the basis of a clinical protocol using a large language model, the system comprising: a service provision module for receiving a clinical protocol from at least one user terminal, generating documents and/or data related to the clinical protocol, and providing the documents and/or data to the at least one user terminal; and a large language model for receiving at least one prompt and information related to the clinical protocol indexed by the service provision module from the service provision module, and providing an output thereof to the service provision module, wherein the service provision module includes: a conversion rule management unit for providing a conversion rule generation function including the at least one prompt to the user terminal; and a conversion execution unit for providing the conversion rule and the clinical protocol to the large language model, and providing documents and/or data output from the large language model to the at least one user terminal in a final output form.

The service provision module may further include a clinical protocol index unit for indexing and retrieving the clinical protocol.

The clinical protocol index unit may index and retrieve the clinical protocol on the basis of the conversion rule selected by the at least one user terminal.

The service provision module may further include an assistant information generation unit for providing assistant information to help the at least one user terminal in generating the conversion rule, wherein the assistant information may include an example of the at least one prompt and a number of prompts for outputting documents and/or data desired to be generated.

The system may further comprise a document management module for managing the documents and/or data related to the clinical protocol, wherein the document management module may provide the at least one user terminal with an editing function for the documents and/or data related to the clinical protocol.

The document management module may confirm whether the documents and/or data related to the clinical protocol are edited by the at least one user terminal, and generates edited data including edited contents of the documents and/or data as learning data when the at least one user terminal edits the documents and/or data related to the clinical protocol, and the large language model may be updated on the basis of the learning data.

The system may further comprise an assistant learning module for learning a plurality of clinical protocols and documents and/or data previously generated in relation to each of the plurality of clinical protocols, and generating the assistant information, wherein the service provision module may further include a clinical protocol index unit for indexing and retrieving the clinical protocol, and the assistant learning module may be updated on the basis of the learning data.

The conversion rule management unit may modify the conversion rule generated by the at least one user terminal in association with the assistant learning module.

The assistant learning module may allow the clinical protocol index unit to reindex the clinical protocol on the basis of the learning data.

To accomplish the object, according to another aspect of the present invention, there is provided a method of generating related documents and data on the basis of a clinical protocol using a large language model, the method performed by a system and comprising the steps of: providing a conversion rule generation function to at least one user terminal; receiving a clinical protocol from the at least one user terminal; providing a conversion rule, including at least one prompt generated through the conversion rule generation function, and the clinical protocol to the large language model; and receiving documents and/or data related to the clinical protocol output from the large language model, converting the documents and/or data into a final output form, and providing the final output form to the at least one user terminal.

The step of providing the clinical protocol to the large language model may include the step of indexing and retrieving the clinical protocol by the system.

The step of indexing and retrieving the clinical protocol may index and retrieve the clinical protocol on the basis of the conversion rule.

The method of generating related documents and data may further comprise the step of providing assistant information to the at least one user terminal to help generation of the conversion rule, wherein the assistant information includes an example of the at least one prompt and a number of prompts for outputting documents and/or data desired to be generated.

The method of generating related documents and data may further comprise the step of providing the at least one user terminal with an editing function for the documents and/or data related to the clinical protocol.

The method of generating related documents and data may further comprise the steps of: confirming whether the documents and/or data related to the clinical protocol are edited by the at least one user terminal; generating edited data including edited contents of the documents and/or data as learning data when the at least one user terminal edits the documents and/or data related to the clinical protocol; and updating the large language model on the basis of the learning data.

The method of generating related documents and data may further comprise the step of learning a plurality of clinical protocols and documents and/or data previously generated in relation to each of the plurality of clinical protocols, and generating the assistant information, wherein the assistant information is updated on the basis of the learning data.

The method of generating related documents and data may further comprise the step of modifying the conversion rule generated by the at least one user terminal on the basis of the learning data.

The method of generating related documents and data may further comprise the step of reindexing the clinical protocol on the basis of the learning data.

Specific details of other embodiments are included in the detailed description and drawings.

### BRIEF DESCRIPTION OF THE DRAWINGS

FIG. 1 is a view showing the network configuration of a system for generating related documents and data on the basis of a clinical protocol using a large language model according to an embodiment of the present invention.
FIG. 2 is a block diagram showing the configuration of a system for generating related documents and data on the basis of a clinical protocol using a large language model according to an embodiment of the present invention.
FIG. 3 is a block diagram showing the detailed configuration of a service provision module according to an embodiment of the present invention.
FIG. 4 is a flowchart illustrating a method of generating related documents and data on the basis of a clinical protocol using a large language model according to an embodiment of the present invention.
FIG. 5 is a flowchart illustrating, as a method of generating related documents and data on the basis of a clinical protocol using a large language model according to an embodiment of the present invention, a method for learning a large language model and/or an assistant learning module for improving accuracy of output results.

### DETAILED DESCRIPTION OF THE PREFERRED EMBODIMENT

The advantages and features of the present invention and a method for achieving them will become clear by referring to the embodiments described below in detail, together with the accompanying drawings. However, the present invention is not limited to the embodiments disclosed below and will be implemented in various different forms. These embodiments are provided only to make the disclosure of the present invention complete and to fully inform those skilled in the art of the present invention of the scope of the present invention, and the present invention is defined by the scope of the claims.

The terms used in this application are only used to describe specific embodiments and are not intended to limit the present invention. Singular expressions include plural expressions unless the context clearly dictates otherwise. It should be understood that terms in this application, such as "comprise" or "have", are intended to designate the presence of features, numbers, steps, operations, components, parts, or combinations thereof described in the specification, and do not preclude in advance the possibility of the presence or addition of one or more other features, numbers, steps, operations, components, parts, or combinations thereof.

Although first, second, and the like are used to describe various components, it goes without saying that these components are not limited by these terms. These terms are used only to distinguish one component from the others. Therefore, it goes without saying that a first component mentioned below may also be a second component within the technical spirit of the present invention.

In a way similar to that the components disclosed in this specification may be implemented as software programming or software elements, embodiments of the present invention may be implemented in a programming or scripting language such as C, C++, Java, assembler, or the like, including various algorithms implemented as combinations of data structures, processes, routines, or other programming components. Functional aspects may be implemented as algorithms executed on one or more processors. In addition, this embodiment may employ conventional techniques for at least one among electronic environment setting, signal processing, and data processing. Terms such as "mechanism," "element," "means," and "component" may be used broadly and are not limited to mechanical and physical components. The terms may include the meaning of a series of software routines in connection with a processor or the like.

Hereinafter, specific embodiments will be described with reference to the accompanying drawings.

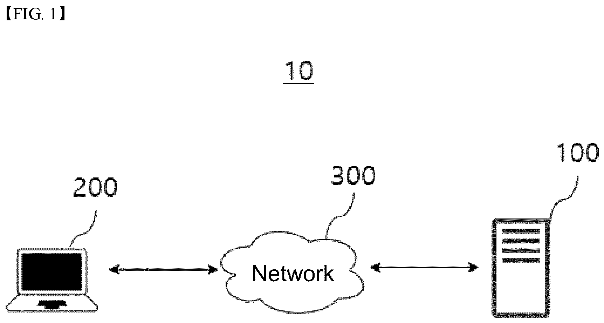

FIG. 1 is a view showing the network configuration of a system for generating related documents and data on the basis of a clinical protocol using a large language model according to an embodiment of the present invention.

Referring to FIG. 1, the network configuration 10 of a document management system according to an embodiment may include a system 100 for generating related documents and data on the basis of a clinical protocol using a large language model, at least one user terminal 200, and a network 300.

The system 100 for generating related documents and data on the basis of a clinical protocol using a large language model (hereinafter, abbreviated as a 'system') may provide a service for generating related documents and data on the basis of a clinical protocol using a large language model to the user terminal 200 through a cloud computing method, and specifically, the service may be provided to the user terminal 200 as Software as a Service (SaaS) through the network 300, but it is not limited thereto.

In some embodiments, the document management system 100 may be provided to the user terminal 200 in various computing methods, such as Platform as a Service (PaaS), Infrastructure as a Service (IaaS), or the like, as well as in the form of Software as a Service. Furthermore, the system 100 may be provided as a software program to be installed and driven in the user terminal 200 according to a traditional manner.

The system 100 may generate documents and/or data related to a clinical protocol requested by the user terminal 200 on the basis of a clinical protocol received from the user terminal 200 and at least one preset conversion rule, and provide the documents and/or data to the user terminal 200.

The documents and/or data related to a clinical protocol may mean documents and/or data generated on the basis of information included in the clinical protocol. For example, the documents and/or data related to a clinical protocol may include an Electronic Case Report Form (eCRF) that will be used in conducting a clinical trial, a clinical trial schedule, documents or information (e.g., consent form, subject information, information related to compensation, etc.) to be provided to a subject, a clinical trial monitoring report form, a clinical trial result report form, and the like, but they are not limited thereto.

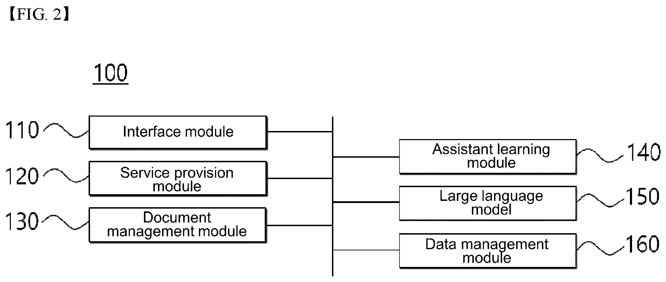

The system 100 may provide the user terminal 200 with an editing function for the related documents and/or data. The system 100 may extract contents edited by the user terminal 200 to improve accuracy of the documents and/or data related to a clinical protocol, and may improve an artificial intelligence model used to generate the documents and/or data related to a clinical protocol on the basis of the edited contents. A detailed description of the system 100 will be provided below with reference to FIG. 2.

The user terminal 200 may be a device that may access the document management system 100 through a wired or wireless communication network such as the Internet and/or an intranet. For example, the user terminal 200 may be a mobile terminal, such as a laptop computer, a handheld device, a smart phone, a tablet PC, or the like, a desktop computer, or any device that uses such devices or is directly or indirectly connected thereto.

The user terminal 200 may access an application, an application program, and/or a website provided by the system 100 and drive a service provided by the system 100. In some embodiments, each of a plurality of user terminals 200 may be assigned with an identification number by the system 100 and stored and managed in association with information on each of a plurality of users, but it is not limited thereto.

The network 300 is a communication network in which the system 100 and the user terminal 200 communicate with each other, and may be configured regardless of a communication mode. For example, although the communication network 400 may be configured as various communication networks such as Personal Area Network (PAN), Local Area Network (LAN), Metropolitan Area Network (MAN), and Wide Area Network (WAN), it is not limited thereto.

FIG. 2 is a block diagram showing the configuration of a system for generating related documents and data on the basis of a clinical protocol using a large language model according to an embodiment of the present invention.

Referring to FIG. 2, the system 100 according to an embodiment of the present invention may include an interface module 110, a service provision module 120, a document management module 130, an assistant learning module 140, a large language model 150, and a data management module 160.

The interface module 110 may perform a function of controlling the overall operation of each component constituting the system 100. For example, the interface module 110 may perform a function of communicating, linking, and interconnecting data between the components. The interface module 110 may perform various software maintenance functions, such as updating the function of each component of the system 100 under the control of a manager terminal (not shown) that maintains and repairs the system 100, or controlling the access right of the user terminal 200.

The interface module 110 may control data communication between the system 100 and the user terminal 200 and/or data communication between the plurality of user terminals 200. The interface module 110 may perform data communication with the user terminal 200 through the network 300 either in a wired or wireless manner. For example, the interface module 110 may support the data communication function between the system 100 and the user terminal 200 or between the plurality of user terminals 200 through a wired Internet communication method that supports Transmission Control Clinical protocol/Internet Clinical protocol (TCP/IP) or the like or at least any one among various wireless communication methods such as Wideband Code Division Multiple Access (WCMDA), Long Term Evolution (LTE), Wireless Broadband Internet (WiBro), and Wireless Fidelity (WiFi).

The interface module 110 may provide an application, an application program, and/or a website to the user terminal 200 to provide the service provided by the system 100. The interface module 110 may provide a document editing function to the user terminal 200 in association with the document management module 130.

The service provision module 120 may provide documents and/or data related to a clinical protocol to the user terminal 200 in association with the large language model 150. For example, the service provision module 120 may generate documents and/or data related to a clinical protocol on the basis of the clinical protocol received from the user terminal 200 and at least one preset conversion rule, and provide the documents and/or data to the user terminal 200.

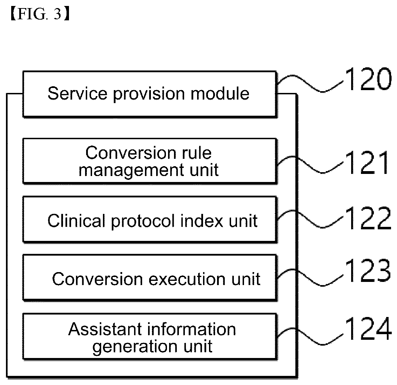

FIG. 3 is a block diagram showing the detailed configuration of a service provision module according to an embodiment of the present invention. Referring to FIG. 3, the service provision module 120 according to an embodiment may include a conversion rule management unit 121, a clinical protocol index unit 122, a conversion execution unit 123, and an assistant information generation unit 124.

The conversion rule management unit 121 may provide the user terminal 200 with a function of generating a conversion rule for deriving documents and/or data desired to be generated on the basis of an input clinical protocol. Here, the conversion rule may include at least one prompt input into the large language model 150 to generate documents and/or data related to a clinical protocol on the basis of information included in the clinical protocol.

The conversion rule management unit 121 may store and manage at least one conversion rule set by the user terminal 200. As a non-limiting example, the conversion rule management unit 121 may store and manage conversion rules on the basis of the identification number or user information of each of the plurality of user terminals 200 in association with the data management module 160. In addition, in some embodiments, the conversion rule management unit 121 may store and manage the conversion rules for each of the plurality of user terminals 200 set as one work group.

The conversion rule management unit 121 may modify at least one conversion rule in association with the assistant learning module 140. For example, the conversion rule management unit 121 may modify a conversion rule generated by the user terminal 200, i.e., at least one prompt included in the conversion rule. As a specific example, as described below, when the user terminal 200 requests generation of documents and/or data related to a clinical protocol using any one conversion rule through the conversion execution unit 123 and edits the generated documents and/or data, the conversion rule management unit 121 may modify at least one prompt included in any one conversion rule so that the edited contents may be reflected.

The clinical protocol index unit 122 may index and retrieve a clinical protocol input by the user terminal 200. The clinical protocol index unit 122 may index the clinical protocol uploaded by the user terminal 200 to extract contents needed for generating documents and/or data related to the clinical protocol by utilizing a large language model.

The clinical protocol index unit 122 may index the clinical protocol by including a process of embedding contents so that the large language model 150 may utilize the contents included in the clinical protocol. As a non-limiting example, although the clinical protocol index unit 122 may index information included in the clinical protocol on the basis of the subject, table of contents, or the like of the contents, it is not limited thereto. The clinical protocol index unit 122 may index the information included in the clinical protocol according to a method understood by those skilled in the art.

In some embodiments, the clinical protocol index unit 122 may index and retrieve the clinical protocol on the basis of a conversion rule selected by the user terminal 200 while uploading the clinical protocol. For example, the clinical protocol index unit 122 may confirm the conversion rule selected by the user terminal 200 and index and retrieve the clinical protocol to extract only the information related to the selected conversion rule.

As a specific example, the Electronic Case Report Form (eCRF) is one of the documents that can be configured on the basis of the contents written in the clinical protocol, and is generally configured to include corresponding contents in the synopsis. Accordingly, when the conversion rule selected by the user terminal 200 corresponds to a conversion rule set to generate an Electronic Case Report Form on the basis of the information included in the clinical protocol, the clinical protocol index unit 122 may retrieve only the information for generating the Electronic Case Report Form among the various contents included in the clinical protocol.

The conversion execution unit 123 may generate documents and/or data related to a clinical protocol on the basis of the conversion rule selected by the user terminal 200 and the information included in the clinical protocol indexed by the clinical protocol index unit 122.

For example, the conversion execution unit 123 may provide at least one prompt included in the conversion rule and information related to the indexed clinical protocol as an input of the large language model 150, and receive an output of the large language model 150. The conversion execution unit 123 may provide the received output of the large language model 150 to the user terminal 200 as a final result or may convert the output of the large language model 150 into a final result form and provide it to the user terminal 200 as needed.

As a more specific example, a conversion rule according to an embodiment may be set to generate the Electronic Case Report Form on the basis of the contents described in the clinical protocol, and a specific conversion rule generated by the user terminal 200 may be as shown below in Embodiment 1.

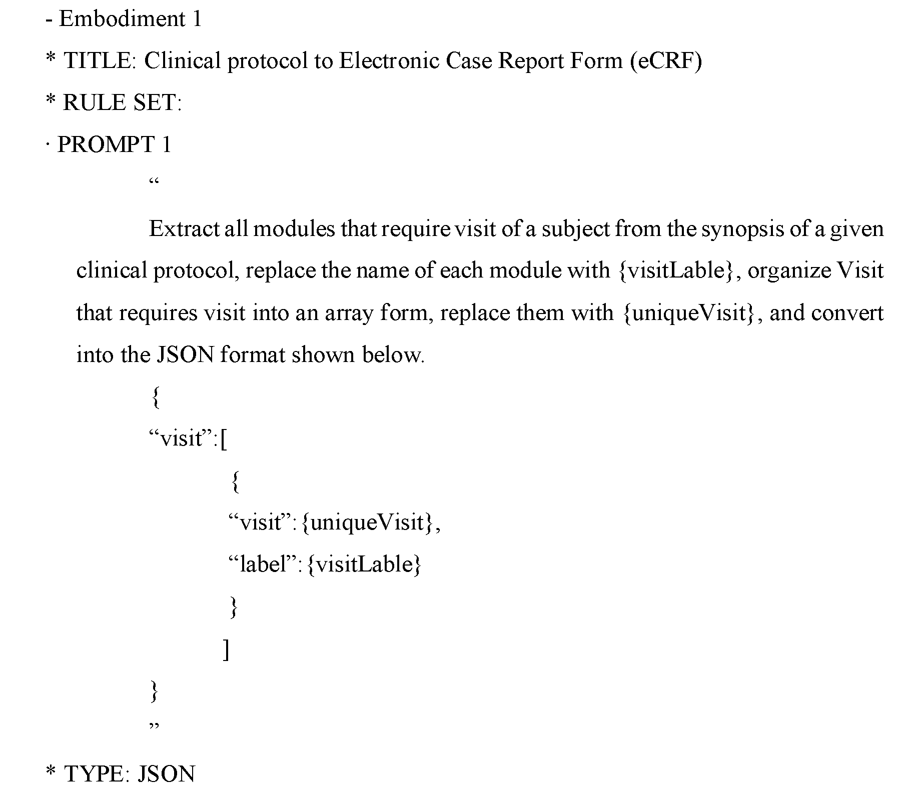

As another specific example, a conversion rule according to an embodiment may be set to generate a clinical trial schedule (Project Management) document on the basis of the contents described in the clinical protocol, and a specific conversion rule written by the user terminal 200 may be as shown below in Embodiment 2.

```
          - Embodiment 2
          * TITLE: Clinical protocol to clinical trial schedule (project schedule)
          * RULE SET:
          · PROMPT 1
             "
                    When the indication is an anticancer drug in a given clinical protocol, add
             an item by setting 10 days as the Working Day of "eCRF Design", otherwise 7 days.
             "
          · PROMPT 2
             "
                    Add an item by setting the number of clinical trial progress modules of a
             given clinical protocol as the Working Day of the "Edit Confirm Programming"
             item.
             "
          · PROMPT 3
             "
                    Add added items as items of Task, Working Day, Start Day, and End Day,
             respectively. Set the Start Date of the first item to 2024/01/01, and starting from
             the second item, add Working Day in the End Date of the previous item to create a
             table as specified.
             "
          * TYPE: DOCX
```

In the cases of embodiments 1 and 2 described above, each conversion execution unit 123 may input at least one prompt included in each conversion rule and contents included in the clinical protocol indexed by the clinical protocol index unit 122 into the large language model 150. When the large language model 150 outputs a result in the JSON format specified as the conversion rule through embodiment 1, the conversion execution unit 123 may provide the output result to the user terminal 200.

When the result output from the large language model 150 through embodiment 2 described above is in the form of an MS-Word document (DOCX) set by the conversion rule, the conversion execution unit 123 provides the document to the user terminal 200. However, when the result output from the large language model 150 is in a form other than an MS-Word document, the conversion execution unit 123 may convert the document into the MS-Word document form and provide it to the user terminal 200, but it is not limited thereto.

The assistant information generation unit 124 may generate and provide assistant information in association with the assistant learning module 140 to help the user terminal 200 in generating a conversion rule. For example, when the user terminal 200 requests generation of a conversion rule through the conversion rule management unit 121, the assistant information generation unit 124 may provide the user terminal 200 with a query (or a selectable user interface (UI)) asking a type of documents and/or data desired to be generated by the conversion rule in association with the conversion rule management unit 121, and provide assistant information that is helpful in writing a prompt in correspondence to the response of the user terminal 200.

As a specific example, the assistant information may include examples of prompts for outputting documents and/or data desired to be generated, the number of prompts required to generate the documents, required items to be included in the documents, and the like, but it is not limited thereto.

In addition, the assistant information generation unit 124 may provide the assistant information while the user terminal 200 is writing a prompt for generating a conversion rule or after writing the prompt. For example, the assistant information generation unit 124 may determine whether the prompt input by the user terminal 200 is suitable for the documents and/or data desired to be generated, and when it is determined that the prompt is not suitable or requires modification to derive more accurate results, the assistant information generation unit 124 may provide the user terminal 200 with assistant information including contents to be modified.

Referring to FIG. 2 again, the document management module 130 may manage documents and/or data related to a clinical protocol generated by the service provision module 120. In addition, the document management module 130 may store the documents and/or data generated by the service provision module 120 through the data management module 160. For example, the document management module 130 may manage and store the documents and/or data generated by the service provision module 120 on the basis of the identification number or user information of each of the plurality of user terminals 200. As another example, the document management module 130 may store and manage the documents and/or data related to a clinical protocol for each of the plurality of user terminals 200 set as one work group.

The document management module 130 may provide the user terminal 200 with an editing function for the documents and/or data related to a clinical protocol in association with an external document editing program corresponding to various document formats. For example, the document management module 130 may provide the user terminal 200 with an editing function for various documents in real time on the network 300 through the interface module 110, but it is not limited thereto.

In some embodiments, the document management module 130 may be implemented to include a document editing program having its own document format, and in this case, the document management module 130 may provide the user terminal 200 with its own editing function for the corresponding document format.

The document management module 130 may confirm whether the documents and/or data related to a clinical protocol are edited by the user terminal 200. For example, when the document management module 130 provide a document editing function to the user terminal 200 in association with an external document editing program, the document management module 130 may monitor the contents of the documents and/or data related to a clinical protocol edited by the user terminal 200 in real time. As another example, the document management module 130 may confirm whether the documents and/or data are edited by the user terminal 200 by comparing the documents and/or data related to a clinical protocol provided to the user terminal 200 with the documents and/or data related to a clinical protocol edited by the user terminal 200.

In addition, when the user terminal 200 edits the documents and/or data related to a clinical protocol, the document management module 130 may generate, manage, and store the edited data including edited contents of the documents and/or data as learning data. The document management module 130 may manage and store the learning data in association with the corresponding documents and/or data related to a clinical protocol. The document management module 130 may provide the learning data to the assistant learning module 140.

The assistant learning module 140 may automatically generate prompts to be included when a conversion rule is generated. For example, the assistant learning module 140 may learn a plurality of clinical protocols and documents and/or data related to each of the plurality of previously generated clinical protocols, and generate assistant information corresponding to documents and/or data desired to be generated in the conversion rule. Here, the assistant information may be the same as the assistant information described in relation to the assistant information generation unit 124.

The assistant learning module 140 may update the assistant information by reflecting the learning data generated and provided by the document management module 130. In this case, the assistant learning module 140 may update the assistant information for each of the plurality of user terminals 200 or for each of the plurality of user terminals 200 set as a certain work group. In addition, the assistant learning module 140 may allow the conversion rule management unit 121 to modify the previously generated conversion rule by using the updated assistant information.

In addition, the assistant learning module 140 may allow the clinical protocol index unit 122 to reindex the clinical protocol on the basis of the learning data. For example, when the assistant learning module 140 determines that editing of the documents and/or data is performed by the user terminal 200 in a method of embedding contents, i.e., an indexing method, of the clinical protocol index unit 122, the assistant learning module 140 may allow the clinical protocol index unit 122 to automatically reindex the clinical protocol on the basis of the learning data.

The assistant information generation unit 124 and/or the assistant learning module 140 may be implemented as an artificial intelligence (AI) model for providing the functions described above to the user terminal 200. The assistant information generation unit 124 and/or the assistant learning module 140 may be implemented in various forms of AI models, as is understood by those skilled in the art. Although it is exemplified in the present specification that the assistant information generation unit 124 and the assistant learning module 140 are implemented as separate components, it is not limited thereto, and it goes without saying that the assistant information generation unit 124 and the assistant learning module 140 may be implemented as a single AI model in other embodiments.

The large language model 150 is an extremely large deep learning model previously trained on the basis of a large amount of data, and may provide answers to input prompts. According to the present invention, the large language model 150 may output results on the basis of at least one prompt received from the service provision module 120 and information related to an indexed clinical protocol needed to provide answers to the prompts. The large language model 150 may use various models understood by those skilled in the art.

**In** addition, in some embodiments, the large language model 150 according to the present invention may be implemented as an artificial intelligence model obtained by additionally training an existing large language model on the basis of a plurality of clinical protocols, documents and/or data related to the plurality of clinical protocols, and further, training data generated by the document management module 130.

The data management module 160 may manage and store data generated or collected through the system 100. The data management module 160 may store the data in a separate external database (not shown), but it is not limited thereto, and it may be implemented to include a database of its own.

The data management module 160 may store and manage information on a plurality of user terminals 200 or users. For example, the data management module 160 may store and manage personal information (e.g., ID, password, biometric authentication information, etc.) of each user (or user terminal 200) for accessing the system 100, work authority information of each user, log records, and the like.

The data management module 160 may store and manage service-related data including data generated by the system 100, such as a plurality of conversion rules generated by the conversion rule management unit 121, a plurality of clinical protocols uploaded by the plurality of user terminals 200, documents and/or data each associated with the plurality of clinical protocols generated by the conversion execution unit 123, and assistant information generated by the assistant information generation unit 124.

When the data management module 160 stores and manages data generated by the system 100, it may manage and store each of the generated data on the basis of the identification number or user information of each of the plurality of user terminals 200. In addition, the data management module 160 may also store and manage the data generated by the system 100 for each of the plurality of user terminals 200 set as one work group.

As described above, the system 100 for generating related documents and data on the basis of a clinical protocol using a large language model according to an embodiment may reduce the time and cost required for generating documents and data needed for conducting a clinical trial by generating various documents and data needed to conduct a clinical trial on the basis of previously generated clinical protocols.

In addition, the system 100 according to the present invention may avoid the problem of hallucination, which may occur as general data is learned, and enhance accuracy of documents and/or data of final results by providing contents of the indexed clinical protocol, as well as prompts, to the large language model in generating the documents and data.

Furthermore, the system 100 according to the present invention may be advantageous in providing a final result (i.e., documents and/or data related to a clinical protocol) optimized for each user terminal 200 by modifying existing conversion rules on the basis of editing contents of the user terminal 200 for the documents and/or data generated by the system 100 and providing assistant information to the user terminal 200.

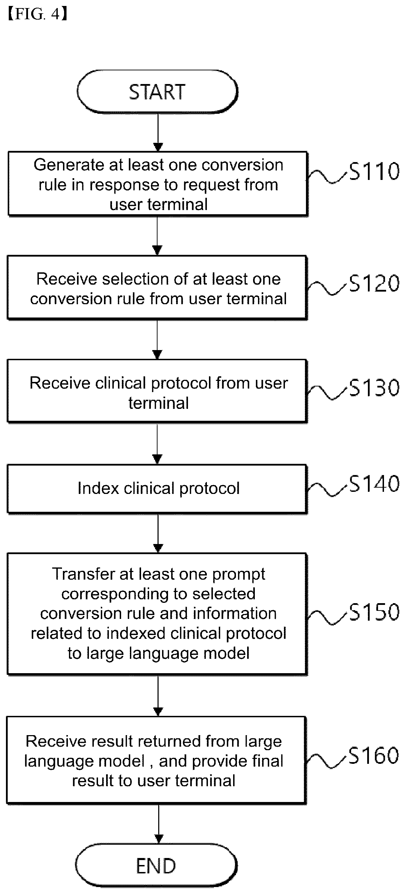

FIG. 4 is a flowchart illustrating a method of generating related documents and data on the basis of a clinical protocol using a large language model according to an embodiment of the present invention.

Referring to FIGS. 1 to 4, the service provision module 120 may generate a conversion rule in response to a request from the user terminal 200 (S110).

For example, the service provision module 120 may provide the user terminal 200 with a function of generating a conversion rule for deriving documents and/or data desired to be generated on the basis of an input clinical protocol. Here, the conversion rule may include at least one prompt input into the large language model 150 to generate documents and/or data related to a clinical protocol on the basis of information included in the clinical protocol.

The service provision module 120 may store and manage at least one conversion rule set by the user terminal 200. As a non-limiting example, the service provision module 120 may store and manage conversion rules on the basis of the identification number or user information of each of the plurality of user terminals 200 in association with the data management module 160. In addition, in some embodiments, the service provision module 120 may store and manage the conversion rules for each of the plurality of user terminals 200 set as one work group.

In addition, in providing a conversion rule generation function to the user terminal 200 in association with the assistant learning module 140, the service provision module 120 may generate and provide assistant information to help the user terminal 200 in generating a conversion rule.

For example, when the user terminal 200 requests generation of a conversion rule through the conversion rule management unit 121, the assistant information generation unit 124 may provide the user terminal 200 with a query (or a selectable user interface (UI)) asking a type of documents and/or data desired to be generated by the conversion rule in association with the conversion rule management unit 121, and provide assistant information that is helpful in writing a prompt in correspondence to the response of the user terminal 200.

As a specific example, the assistant information may include examples of prompts for outputting documents and/or data desired to be generated, the number of prompts required to generate the documents, required items to be included in the documents, and the like, but it is not limited thereto.

In addition, the assistant information generation unit 124 may provide the assistant information while the user terminal 200 is writing a prompt for generating a conversion rule or after writing the prompt. For example, the assistant information generation unit 124 may determine whether the prompt input by the user terminal 200 is suitable for the documents and/or data desired to be generated, and when it is determined that the prompt is not suitable or requires modification to derive more accurate results, the assistant information generation unit 124 may provide the user terminal 200 with assistant information including contents to be modified.

Thereafter, the service provision module 120 may receive a selection of at least one conversion rule from the user terminal 200 (S120) and receive a clinical protocol (S130).

The step of receiving a selection of at least one conversion rule from the user terminal 200 by the service provision module 120 (S120) and the step of receiving a clinical protocol may be performed substantially at the same time, but it is not limited thereto (S130).

Next, the service provision module 120 may index the clinical protocol (S140). The service provision module 120 may index the clinical protocol uploaded by the user terminal 200 to extract contents needed for generating documents and/or data related to the clinical protocol by utilizing a large language model.

For example, although the service provision module 120 may index information included in the clinical protocol on the basis of the subject, table of contents, or the like of the contents, it is not limited thereto. The service provision module 120 may index the information included in the clinical protocol according to a method understood by those skilled in the art.

In some embodiments, the service provision module 120 may index the clinical protocol on the basis of a conversion rule selected by the user terminal 200 while uploading the clinical protocol. For example, the service provision module 120 may confirm the conversion rule selected by the user terminal 200 and index the clinical protocol to extract only the information related to the selected conversion rule.

As a specific example, the service provision module 120 is one of the documents that can be configured on the basis of the contents written in the clinical protocol, and is generally configured to include corresponding contents in the synopsis. Accordingly, when the conversion rule selected by the user terminal 200 corresponds to a conversion rule set to generate an Electronic Case Report Form on the basis of the information included in the clinical protocol, the system 100 may index only the information for generating the Electronic Case Report Form among the various contents included in the clinical protocol.

Then, the service provision module 120 may input at least one prompt corresponding to the selected conversion rule and information related to the indexed clinical protocol into the large language model 150 (S150), and provide a final result to the user terminal 200 after receiving the result returned from the large language model (S160).

That is, the service provision module 120 may generate documents and/or data related to the clinical protocol on the basis of the conversion rule selected by the user terminal 200 and the information included in the indexed clinical protocol.

For example, the service provision module 120 may provide at least one prompt included in the conversion rule and information related to the indexed clinical protocol as an input of the large language model 150, and receive an output of the large language model 150. The service provision module 120 may provide the received output of the large language model 150 to the user terminal 200 as a final result or may convert the output of the large language model 150 into a final result form and provide it to the user terminal 200 as needed.

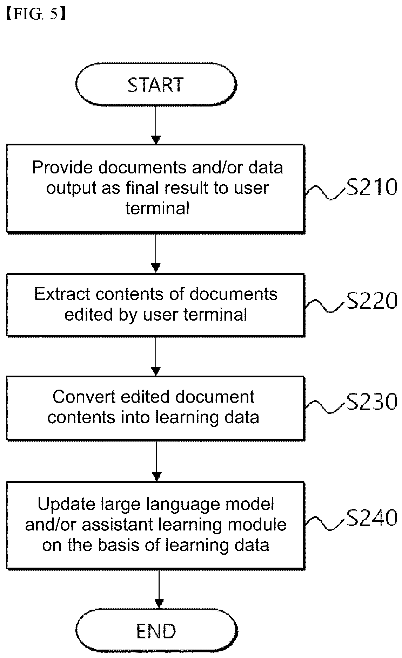

FIG. 5 is a flowchart illustrating, as a method of generating related documents and data on the basis of a clinical protocol using a large language model according to an embodiment of the present invention, a method for learning a large language model and/or an assistant learning module for improving accuracy of output results.

Referring to FIGS. 1 to 5, the system 100 may provide documents and/or data related to a clinical protocol output as a final result through steps S110 to S150 of FIG. 4 to the user terminal 200 (S210), and extract the contents of the documents and/or data edited by the user terminal 200 (S220).

To this end, the step of extracting the contents of the documents and/or data edited by the user terminal 200 by the system 100 (S220) may include the step of confirming whether the documents and/or data has been edited by the user terminal 200.

For example, the document management module 130 may provide the user terminal 200 with an editing function for the documents and/or data related to a clinical protocol in association with an external document editing program corresponding to various document formats. The document management module 130 may provide the user terminal 200 with an editing function for various documents in real time on the network 300 through the interface module 110, but it is not limited thereto.

In another embodiment, the document management module 130 may be implemented to include a document editing program having its own document format, and in this case, the document management module 130 may provide the user terminal 200 with its own editing function for the corresponding document format.

The document management module 130 may confirm whether the documents and/or data related to a clinical protocol are edited by the user terminal 200. For example, when the document management module 130 provide a document editing function to the user terminal 200 in association with an external document editing program, the document management module 130 may monitor the contents of the documents and/or data related to a clinical protocol edited by the user terminal 200 in real time. As another example, the document management module 130 may confirm whether the documents and/or data are edited by the user terminal 200 by comparing the documents and/or data related to a clinical protocol provided to the user terminal 200 with the documents and/or data related to a clinical protocol edited by the user terminal 200.

When it is confirmed that the user terminal 200 has edited the documents and/or data related to a clinical protocol, the system 100 may extract the contents of the documents and/or data edited by the user terminal 200 (S220), and convert the edited document contents into learning data (S230).

For example, when the user terminal 200 edits the documents and/or data related to a clinical protocol, the document management module 130 may generate, manage, and store the edited data including edited contents of the documents and/or data as learning data. The document management module 130 may manage and store the learning data in association with the corresponding documents and/or data related to a clinical protocol. The document management module 130 may provide the learning data to the assistant learning module 140.

Next, the system 100 may update the large language model 150 and/or the assistant learning module 140 on the basis of the learning data (S240).

First, the system 100 may update the large language model 150 on the basis of the learning data that reflects the contents edited by the user terminal 200 so that the large language model 150 may derive more accurate results in generating documents and/or data related to a clinical protocol according to the present invention.

In addition, the assistant learning module 140 according to the present invention may automatically generate prompts to be included when a conversion rule is generated. For example, the assistant learning module 140 may learn a plurality of clinical protocols and documents and/or data related to each of the plurality of previously generated clinical protocols, and generate assistant information corresponding to documents and/or data desired to be generated in the conversion rule.

The assistant learning module 140 like this may update the assistant information by reflecting the learning data generated and provided by the document management module 130. In this case, the assistant learning module 140 may update the assistant information for each of the plurality of user terminals 200 or for each of the plurality of user terminals 200 set as a certain work group. In addition, the assistant learning module 140 may allow the conversion rule management unit 121 to modify the previously generated conversion rule by using the updated assistant information.

As described above, the method of generating related documents and data on the basis of a clinical protocol using a large language model according to an embodiment of the present invention may reduce the time and cost required for generating documents and data needed for conducting a clinical trial by generating various documents and data needed to conduct a clinical trial on the basis of previously generated clinical protocols.

In addition, the method of generating related documents and data on the basis of a clinical protocol using a large language model according to an embodiment of the present invention may avoid the problem of hallucination, which may occur as general data is learned, and enhance accuracy of documents and/or data of final results by providing contents of the indexed clinical protocol, as well as prompts, to the large language model in generating the documents and data.

Furthermore, the method of generating related documents and data on the basis of a clinical protocol using a large language model according to the present invention may be advantageous in providing a final result (i.e., documents and/or data related to a clinical protocol) optimized for each user terminal 200 by modifying existing conversion rules on the basis of editing contents of the user terminal 200 for the documents and/or data generated by the system 100 and providing assistant information to the user terminal 200.

The features of the various embodiments of the present invention may be partially or entirely assembled or combined with each other, and various interconnections and operations are technically possible, and the embodiments may be implemented to be independent from each other or together as a related relationship.

Although the embodiments of the present invention have been described above with reference to the accompanying drawings, those skilled in the art will understand that the present invention may be implemented in other specific forms without changing the technical spirits or essential features of the present invention. These embodiments are not intended to limit the present invention and are merely illustrative, and should be considered from an illustrative perspective rather than a restrictive perspective. Although specific terms are used in this specification, they are used only for the purpose of explaining the concept of the present invention and are not used to limit the meaning or scope of the present invention described in the claims. Therefore, the embodiments described above should be understood in all respects as illustrative and not restrictive.

The system and method for generating related documents and data on the basis of a clinical protocol using a large language model according to the embodiments of the present invention may reduce the time and cost required for generating documents and data needed for conducting a clinical trial by generating various documents and data needed to conduct a clinical trial on the basis of previously generated clinical protocols.

In addition, the present invention may avoid the problem of hallucination, which may occur as general data is learned, and enhance accuracy of documents and/or data of final results by providing contents of the indexed clinical protocol, as well as prompts, to the large language model in generating the documents and data.

Furthermore, the present invention may be advantageous in providing a final result (i.e., documents and/or data related to a clinical protocol) optimized for each user terminal by modifying existing conversion rules on the basis of editing contents of the user terminal for the documents and/or data generated by the system and providing assistant information to the user terminal.

The effects according to the embodiments of the present invention are not limited to the content exemplified above, and further various effects are included in this specification.

### DESCRIPTION OF SYMBOLS

| | | | |
|---|---|---|---|
| 100: | System for generating related documents and data on the basis of clinical protocol using large language model | | |
| 200: | User terminal | 300: | Network |
| 110: | Interface module | 120: | Service provision module |
| 130: | Document management module | 140: | Assistant learning module |
| 150: | Large language model | 160: | Data management module |
| 121: | Conversion rule management unit | | |
| 122: | Clinical protocol index unit | | |
| 123: | Conversion execution unit | | |
| 124: | Assistant information generation unit | | |

## Claims

1. A system for generating related documents and data on the basis of a clinical protocol using a large language model, the system comprising:
a service provision module for receiving a clinical protocol from at least one user terminal, generating documents and/or data related to the clinical protocol, and providing the documents and/or data to the at least one user terminal; and
a large language model for receiving at least one prompt and information related to the clinical protocol indexed by the service provision module from the service provision module, and providing an output thereof to the service provision module, wherein
the service provision module includes:
a conversion rule management unit for providing a conversion rule generation function including the at least one prompt to the user terminal; and
a conversion execution unit for providing the conversion rule and the clinical protocol to the large language model, and providing documents and/or data output from the large language model to the at least one user terminal in a final output form.

2. The system according to claim 1, wherein the service provision module further includes a clinical protocol index unit for indexing and retrieving the clinical protocol.

3. The system according to claim 2, wherein the clinical protocol index unit indexes and retrieves the clinical protocol on the basis of the conversion rule selected by the at least one user terminal.

4. The system according to claim 1, wherein the service provision module further includes an assistant information generation unit for providing assistant information to help the at least one user terminal in generating the conversion rule, wherein the assistant information includes an example of the at least one prompt and a number of prompts for outputting documents and/or data desired to be generated.

5. The system according to claim 4, further comprising a document management module for managing the documents and/or data related to the clinical protocol, wherein the document management module provides the at least one user terminal with an editing function for the documents and/or data related to the clinical protocol.

6. The system according to claim 5, wherein the document management module confirms whether the documents and/or data related to the clinical protocol are edited by the at least one user terminal, and generates edited data including edited contents of the documents and/or data as learning data when the at least one user terminal edits the documents and/or data related to the clinical protocol, and the large language model is updated on the basis of the learning data.

7. The system according to claim 6, further comprising an assistant learning module for learning a plurality of clinical protocols and documents and/or data previously generated in relation to each of the plurality of clinical protocols, and generating the assistant information, wherein the service provision module further includes a clinical protocol index unit for indexing and retrieving the clinical protocol, and the assistant learning module is updated on the basis of the learning data.

8. The system according to claim 7, wherein the conversion rule management unit modifies the conversion rule generated by the at least one user terminal in association with the assistant learning module.

9. The system according to claim 7, wherein the assistant learning module allows the clinical protocol index unit to reindex the clinical protocol on the basis of the learning data.

10. A method of generating related documents and data on the basis of a clinical protocol using a large language model, the method performed by a system and comprising the steps of:
providing a conversion rule generation function to at least one user terminal;
receiving a clinical protocol from the at least one user terminal;
providing a conversion rule, including at least one prompt generated through the conversion rule generation function, and the clinical protocol to the large language model; and
receiving documents and/or data related to the clinical protocol output from the large language model, converting the documents and/or data into a final output form, and providing the final output form to the at least one user terminal.

11. The method according to claim 10, wherein the step of providing the clinical protocol to the large language model includes the step of indexing and retrieving the clinical protocol by the system.

12. The method according to claim 11, wherein the step of indexing and retrieving the clinical protocol indexes and retrieves the clinical protocol on the basis of the conversion rule.

13. The method according to claim 10, further comprising the step of providing assistant information to the at least one user terminal to help generation of the conversion rule, wherein the assistant information includes an example of the at least one prompt and a number of prompts for outputting documents and/or data desired to be generated.

14. The method according to claim 13, further comprising the step of providing the at least one user terminal with an editing function for the documents and/or data related to the clinical protocol.

15. The method according to claim 14, further comprising the steps of:
confirming whether the documents and/or data related to the clinical protocol are edited by the at least one user terminal;
generating edited data including edited contents of the documents and/or data as learning data when the at least one user terminal edits the documents and/or data related to the clinical protocol; and
updating the large language model on the basis of the learning data.

16. The method according to claim 15, further comprising the step of learning a plurality of clinical protocols and documents and/or data previously generated in relation to each of the plurality of clinical protocols, and generating the assistant information, wherein the assistant information is updated on the basis of the learning data.

17. The method according to claim 16, further comprising the step of modifying the conversion rule generated by the at least one user terminal on the basis of the learning data.

18. The method according to claim 17, further comprising the step of reindexing the clinical protocol on the basis of the learning data.
